**Europäisches Patentamt**

**European Patent Office**

**Office européen des brevets**

(19)

(11) Publication number: **0 323 522**
**A1**

(12)

# EUROPEAN PATENT APPLICATION

published in accordance with Art. 158(3) EPC

(21) Application number: **88906068.7**

(22) Date of filing: **06.07.88**

Data of the international application taken as a basis:

(86) International application number:
**PCT/JP88/00674**

(87) International publication number:
**WO89/00044 (12.01.89 89/02)**

(51) Int. Cl.,³: **A 61 K 9/08**
**A 61 K 31/715**

(30) Priority: **07.07.87 JP 170210/87**

(43) Date of publication of application:
**12.07.89 Bulletin 89/28**

(84) Designated Contracting States:
**AT BE CH DE FR GB IT LI LU NL SE**

(71) Applicant: **SANTEN PHARMACEUTICAL CO., LTD.**
**9-19, 3-Chome, Shimoshinjo**
**Higashiyodogawa-ku Osaka533(JP)**

(72) Inventor: **IWAO, Jun-ichi**
**7-27, Nogami 4-chome**
**Takarazuka-shi Hyogo 665(JP)**

(72) Inventor: **ISO, Tadashi**
**22-18, Kiyomidai 1-chome**
**Kawachinagano-shi Osaka 586(JP)**

(72) Inventor: **UEMURA, Osamu**
**17-2, Fujishirodai 2-chome**
**Suita-shi Osaka 565(JP)**

(72) Inventor: **KAWASHIMA, Yo-ichi 4-504, 1-Banchi**
**Oharanohigashisakaidanicho 1-chome Nishikyo-ku**
**Kyoto-shi Kyoto 615(JP)**

(74) Representative: **Pearce, Anthony Richmond et al,**
**MARKS & CLERK Alpha Tower Suffolk Street**
**Queensway Birmingham B1 1TT(GB)**

(54) **ARTIFICIAL LACRIMA.**

(57) An artificial lacrima containing sodium hyaluronate and having a pH of 5.5 to 8.0 and an osmotic pressure ratio (to physiological saline) of 0.85 to 1.55, and a drug for treating corneal xerosis which contains sodium hyaluronate as a major component are disclosed.

SPECIFICATION

## TITLE OF THE INVENTION

### ARTIFICIAL TEAR

## FIELD OF THE ART

This invention provides artificial tear and therapeutic agent for corneal xerosis containing sodium hyaluronate which is useful as a tear supplement.

## BACKGROUND OF THE INVENTION

Hyaluronic acid widely exists in various animal organs such as rooster combs and body fluids. Especially, high molecular hyaluronic acid is known to possess an excellent viscoelasticity and water-holding property, and be useful for lubrication of joints, protection of cornea, etc.
Hyaluronic acid can be highly purified as a sodium salt which is used in ocular surgeries.

As the result of our intensive study of an application of sodium hyaluronate to ophthalmic solution, we found that sodium hyaluronate can be applied to artificial tear based on its excellent properties.

1

DISCLOSURE OF THE INVENTION

Sodium hyaluronate is a compound having high molecular weight, which consists of D-glucuronic acid and N-acetyl-D-glucosamine, and possess excellent water-holding and viscoelastic properties.

As shown in the pharmacological test, we found that hyaluronic acid possess a protective effect of eye dryness and a promoting effect of wound healing and is useful for a treatment of corneal xerosis or keratitis, a prevention of eye dryness associated by wearing contact lenses, a promotion of postoperative wound healing of the cornea. As the result of our study, we found that hyaluronic acid can be applicable to artificial tear.

The content of sodium hyaluronate in the artificial tear of this invention should not be restricted, but preferably be between 0.01% and 1%, and the pH of the solution preferably be between 5.5 and 8.0.

According to the conditions of natural human eye, the pH of the artificial tear of this invention is adjusted to 5.5 - 8.0 and the osmotic pressure ratio thereof to saline to 0.85 - 1.55. Furthermore, other active ingredients acceptable in artificial tear can be combined to this artificial tear.

Sodium chloride, potassium chloride, etc. can be used to adjust the osmotic pressure. Dibasic sodium phosphate, monobasic sodium phosphate, hydrochloric acid, sodium hydroxide, etc. can be used to adjust the pH. If necessary, viscous agent such as polyvinyl alcohol or hydroxyethyl cellulose can further be added.

BEST MODE TO MAKE THE INVENTION

Example 1

Formulation 1

| | |
|---|---|
| Sodium hyaluronate | 0.01g |
| Sodium chloride | 0.9g |
| Methyl para-hydroxybenzoate | 0.026g |
| Propyl para-hydroxybenzoate | 0.014g |
| Sterile purified water | q.s. |
| total | 100ml |

Preparation

Sodium chloride (0.9g), methyl para-hydroxybenzoate (0.026g), and propyl p-hydroxybenzoate (0.014g) were dissolved in sterile purified water (80ml) by heating. After cooling to room temperature, sodium hyaluronate (0.1g) was dissolved in the solution. Total volume was adjusted with sterile purified water to 100ml ( pH 6.8 and osmotic pressure ratio 1.0 ).

Example 2

Formulation 2

| | |
|---|---|
| Sodium hyaluronate | 0.5g |
| Dibasic sodium phosphate | 0.1g |
| Sodium chloride | 0.8g |
| Disodium edetate | 0.05g |
| Ethyl para-hydroxybenzoate | 0.025g |
| Butyl para-hydroxybenzoate | 0.015g |
| Diluted hydrochloric acid | q.s. |

3

Sterile purified water        q.s.

   total        100ml

Preparation

Dibasic sodium phosphate (0.1g), sodium chloride (0.8g), disodium edetate (0.05g), ethyl para-hydroxybenzoate (0.025g) and butyl para-hydroxybenzoate (0.015g) were dissolved in sterile purified water (80ml) by heating, and the solution was cooled to room temperature. Sodium hyaluronate (0.5g) was dissolved in the solution and the pH was adjusted with diluted hydrochloride to 6.0. Total volume of the solution was adjusted with sterile purified water to 100ml ( osmotic pressure ratio 1.1 ).

Example 3

Formulation 3

| | |
|---|---|
| Sodium hyaluronate | 1.0g |
| Sodium chloride | 0.9g |
| Dibasic sodium phosphate | 0.1g |
| Methyl para-hydroxybenzoate | 0.026g |
| Propyl para-hydroxybenzoate | 0.014g |
| Diluted hydrochloric acid | q.s. |
| Sterile purified water | q.s. |
| total | 100ml |

Preparation

The solution was prepared in the same manner as in Example 2 and the pH was adjusted with diluted hydrochloric acid to 7.0 ( osmotic pressure ratio 1.2 ).

Example 4

Formulation 4

| | |
|---|---|
| Sodium hyaluronate | 0.1g |
| Sodium chloride | 0.9g |
| β-Cyclodextrine | 0.2g |
| Benzalkonium chloride | 0.005g |
| Diluted hydrochloric acid or sodium hydroxide | q.s. |
| Sterile purified water | q.s. |
| total | 100ml |

Preparation

Sodium chloride (0.9g), β-cyclodextrine (0.2g) and 0.5ml of 1% benzalkonium chloride solution were dissolved in sterile purified water (80 ml). Sodium hyaluronate (0.1g) was dissolved in the solution and the pH was adjusted with diluted hydrochloric acid or sodium hydroxide to 7.5. Total volume was adjusted to 100ml with sterile purified water ( osmotic pressure ratio 1.1 ).

Example 5

Formulation 5

| | |
|---|---|
| Sodium hyaluronate | 1.0g |
| Sodium chloride | 0.8g |
| Dibasic sodium phosphate | q.s. |
| Monobasic sodium phosphate | q.s. |
| Sterile purified water | q.s. |
| total | 100ml |

Preparation

Sodium chloride (0.8g) was dissolved in sterile purified water (80ml) and the pH was adjusted with dibasic sodium phosphate and monobasic sodium phosphate to 7.5. Sodium hyaluronate was dissolved completely in the solution, and the total volume was adjusted with sterile purified water to 100ml ( osmotic pressure ratio 1.1 ).

The following artificial tears were prepared by the same method.

Formulation 6, ( osmotic pressure ratio 1.1 )

| | |
|---|---|
| Sodium hyaluronate | 0.5g |
| Sodium chloride | 0.85g |
| Dibasic sodium phosphate | q.s. |
| Monobasic sodium phosphate | q.s. |
| Sterile purified water | q.s. |
| total | 100ml |

Formulation 7 ( osmotic pressure ratio 1.0 )

| | |
|---|---|
| Sodium hyaluronate | 0.1g |
| Sodium chloride | 0.85g |
| Dibasic sodium phosphate | q.s. |
| Monobasic sodium phosphate | q.s. |
| Sterile purified water | q.s. |
| total | 100ml |

Pharmacological test

We studied protective effect on eye dryness and promoting effect on corneal wound healing of the artificial tear of this invention in rabbits.

1) Protective effect on eye dryness

Rabbits were killed, and their corneas were removed. 50µl of the artificial tear of this invention ( formulation 5 or 6 ) was immediately dropped on the corneal epithelial. Three hours later, the weight of the cornea was measured and expressed as percent against the weight of the cornea immediately after receiving the drops. As a control, the vehicle removing sodium hyaluronate from the artificial tear of the formulation 5 was used.

( Result )

The results are shown in Table 1.

Table 1    Percent weight of cornea 3 hours after dropping

| | cornea weight(%) |
|---|---|
| Vehicle | 27.3 % |
| Formulation 5 | 37.4 % |
| Formulation 6 | 34.2 % |

As shown in table 1, the artificial tears containing sodium hyaluronate significantly suppressed dryness of the cornea compared with the vehicle, and potency of such effect depended on the concentration of sodium hyaluronate.

The above experiment proves that the artificial tear of this invention possess protective effect of dryness of the eye and is useful for a treatment of corneal xerosis, and a prevention of dry eye in contact lens wearer.

2) Wound healing effect of the cornea

As a method of examining wound healing effect of the cornea by a drug, the exfoliation method of the corneal epithelium with n-heptanol is known. According to this method, we examined wound healing effect of the artificial tear of this invention.

The experiment was performed by the method of Cintron et al. ( Ophthalmic Res., 11, 90 (1979)).

After exfoliation of the corneal epithelium of rabbits, the artificial tear of the formulation 7 and its control vehicle were instilled every 6 hours. We measured area of the wound to examine the healing effect.

Experimental Result

Table 2 shows percent area of the wound at each time against the area just before instillation.

Table 2.    Percent area of the wound  after instillation of
            the artificial tear

---------------------------------------------------------------

Time after exfoliation    Untreated    Vehicle    Formulation 7

---------------------------------------------------------------

| Time after exfoliation | Untreated | Vehicle | Formulation 7 |
|---|---|---|---|
| 24 hr | 60.1 % | 59.2 % | 30.8 % |
| 30 hr | 44.2 % | 44.9 % | 19.2 % |
| 42 hr | 25.7 % | 26.0 % | 5.7 % |

---------------------------------------------------------------

As shown in the table 2, we can hardly recognize the difference in the effect between the group treated with vehicle and the untreated group, but, the group treated with the formulation 7 containing sodium hyaluronate decreased the wound area earlier than that of the vehicle and the untreated groups.

The above experiments proved that the artificial tear of this invention promoted wound healing of the cornea and was useful for the treatment of keratitis such as external injury caused by alkali or exfoliation of corneal epithelium and also useful for promoting postoperative healing.

The utility of the artificial tear of this invention was proved by the above two experiments.


UTILITY IN AN INDUSTRY

This invention provides artificial tear which possess such excellent properties as water-holding effect, etc.    Furthermore, the artificial tear of this invention is useful for the treatment of corneal xerosis.

9

WHAT WE CLAIM IS

1. An artificial tear containing sodium hyaluronate, wherein the pH of the solution is 5.5 to 8.0, and the osmotic pressure ratio thereof to saline is 0.85 to 1.55.

2. A therapeutic agent for corneal xerosis which contains sodium hyaluronate as a main ingredient.

# INTERNATIONAL SEARCH REPORT

International Application No    PCT/JP88/00674

**I. CLASSIFICATION OF SUBJECT MATTER** (if several classification symbols apply, indicate all)

According to International Patent Classification (IPC) or to both National Classification and IPC

Int.Cl⁴    A61K9/08, A61K31/715

**II. FIELDS SEARCHED**

Minimum Documentation Searched

| Classification System | Classification Symbols |
|---|---|
| IPC | A61K9/08, A61K31/715 |

Documentation Searched other than Minimum Documentation
to the Extent that such Documents are Included in the Fields Searched

**III. DOCUMENTS CONSIDERED TO BE RELEVANT**

| Category | Citation of Document, with indication, where appropriate, of the relevant passages | Relevant to Claim No. |
|---|---|---|
| X | JP, A, 58-57319 (The Green Cross Corporation) 5 April 1983 (05. 04. 83) Page 1, left column, 15th line from the bottom to left column, 13th line from the bottom, page 2, right column, line 1 to page 2, left column, 13th line from the bottom, page 2, right column, 13th line from the bottom to right column, 11th line from the bottom (Family: none) | 1-2 |
| X | JP, A, 61-233622 (Riken Vitamin Co., Ltd.) 17 October 1986 (17. 10. 86) Page 1, left column, 15th line from the bottom to left column, 10th line from the bottom, page 2, right column, line 4 to right column, line 14, page 2, right column, 2nd line from the bottom to page 3, left column, line 7 (Family: none) | 1-2 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| September 9, 1988 (09. 09. 88) | September 19, 1988 (19. 09. 88) |

| International Searching Authority | Signature of Authorized Officer |
|---|---|
| Japanese Patent Office | |

Form PCT ISA 210 (second sheet) (January 1985)

0323522

FURTHER INFORMATION CONTINUED FROM THE SECOND SHEET

| A. | Invest. Ophthalmol. Visual Sci., Vol. 25 No. 11, (1984), Hammer, Mark E. et al [Viscous corneal protection by sodium hyaluronate, chondroitin sulfate, and methyl cellulose], p.1329-1332 | 1-2 |

V.☐ OBSERVATIONS WHERE CERTAIN CLAIMS WERE FOUND UNSEARCHABLE [10]

This international search report has not been established in respect of certain claims under Article 17(2) (a) for the following reasons

1.☐ Claim numbers........... because they relate to subject matter [12] not required to be searched by this Authority, namely:

2.☐ Claim numbers........... because they relate to parts of the international application that do not comply with the prescribed requirements to such an extent that no meaningful international search can be carried out [13], specifically:

VI.☐ OBSERVATIONS WHERE UNITY OF INVENTION IS LACKING

This International Searching Authority found multiple inventions in this international application as follows:

1.☐ As all required additional search fees were timely paid by the applicant, this international search report covers all searchable claims of the international application

2.☐ As only some of the required additional search fees were timely paid by the applicant, this international search report covers only those claims of the international application for which fees were paid, specifically claims

3.☐ No required additional search fees were timely paid by the applicant. Consequently, this international search report is restricted to the invention first mentioned in the claims, it is covered by claim numbers

4.☐ As all searchable claims could be searched without effort justifying an additional fee, the International Searching Authority did not invite payment of any additional fee.

Remark on Protest

☐ The additional search fees were accompanied by applicant's protest

☐ No protest accompanied the payment of additional search fees